# EUROPÄISCHE PATENTSCHRIFT

(11) **EP 0 738 165 B1**
(45) Veröffentlichungstag und Bekanntmachung des Hinweises auf die Patenterteilung: **06.05.1998**
(21) Anmeldenummer: 94926218.2
(22) Anmeldetag: 29.08.1994
(51) Int. Cl.: A61M 25/00, A61B 17/36, A61N 1/05

(54) **KATHETER ZUR INJEKTION EINES FLUIDS BZW. EINES ARZNEIMITTELS**
CATHETER FOR INJECTING A FLUID OR MEDICAMENT
CATHETER POUR L'INJECTION D'UN FLUIDE OU D'UN MEDICAMENT

(30) Priorität: 10.03.1994 DE 4408108
(43) Veröffentlichungstag der Anmeldung: 23.10.1996
(73) Patentinhaber: Bavaria Medizin Technologie GmbH, D-82234 Wessling/Oberpfaffenhofen (DE)
(72) Erfinder: HÖFLING, Berthold, D-82234 Wessling (DE)
(74) Vertreter: Fleuchaus, Leo, Dipl.-Ing.
(86) Internationale Anmeldenummer: EP9402850
(87) Internationale Veröffentlichungsnummer: WO9524235

(56) Entgegenhaltungen:
- WO-A-92/10142
- DE-A- 4 235 506
- DE-U- 8 913 761
- FR-A- 2 560 052
- GB-A- 2 269 538
- US-A- 4 578 061
- US-A- 4 940 064

## Beschreibung

Die Erfindung betrifft einen Katheter zur Injektion eines Fluids bzw. eines Arzneimittels in Hohlorgane und Körperhöhlen, insbesondere in Koronargefäße und Arterien mit einer in Arterien einführbaren Katheterspitze und einem Katheterschaft, mehreren in der Katheterspitze verschiebbar angeordneten Injektionsnadeln, wobei die Nadelspitzen in einer eingezogenen Position innerhalb der Katheterspitze liegen und für die Applikation des Fluids oder Arzneimittels in einer herausgeschobenen Position aus der Katheterspitze herausstehen, einer am extrakorporalen Ende angebrachten Betätigungsvorrichtung, mit in der Katheterspitze vorgesehenen Öffnungen mit Nadelführungen, die sowohl einen nach seitlich als auch nach vorne gerichteten Austritt der Injektionsnadeln zulassen, wobei der Katheterschaft am einen Teil der Betätigungsvorrichtung befestigt ist und die Katheterspitze mit einem anderen Teil der Betätigungsvorrichtung verbunden ist, wodurch eine oder mehrere der parallel geführten Injektionsnadeln beim Betätigen der Betätigungsvorrichtung von der zurückgezogenen in die nach außen vorstehende Position durch eine relative Verschiebung der Katheterspitze und der Injektionsnadeln zueinander bewegbar sind.

Durch die US-PS 3 598 119 ist ein medizinisches Gerät zum Verabreichen eines Anästhetikums bekannt, bei dem über ein koaxiales Lumen einerseits eine Injektionnadel im inneren Lumen unter die Hautoberfläche geführt wird, und andererseits eine im distalen Bereich angeordnete Blase über das koaxiale Außenvolumen aufblasbar ist, um die Nadelspitze unter der Haut zu fixieren.

Durch das deutsche Gebrauchsmuster G 89 13 761 ist eine endoskopische Injektionsvorrichtung für einen Fibrinkleber bekannt, bei der drei ineinander koaxial geführte Schläuche mit einer Zuführvorrichtung versehen sind, wobei der äußere Schlauch als Führung für die beiden Innenschläuche dient, durch welche ein Zweikomponenten-Fibrinkleber zugeführt und nach Vermischung im distalen Bereich über eine an einem der koaxialen Schläuche befestigten Injektionsnadel appliziert wird.

Durch die WO 92 10 142 ist ein Katheter bekannt, bei dem in einem mehrlumigen Katheterschaft Nadeln längs verschiebbar sind, die über nach außen führende Öffnungen in der Katheterspitze in das umgebende Gewebe gestoßen werden können, um durch diese Nadeln faseroptische Elemente bzw. thermische Meßelemente in dem Gewebe zu plazieren. Die hohlen Nadeln sind ferner auch zum Einführen von Vorrichtungen zum Spülen und Absaugen geeignet.

Durch die US-PS 4 578 061 ist ein Katheter bekannt, um mit Hilfe einer ausfahrbaren Injektionsnadel in eine Vene oder Arterie eine Flüssigkeit zu injizieren. Die Injektionsnadel ist am vorderen Ende des Katheters in Längsrichtung verschiebbar, wobei die durch das Längslumen des Katheters zugeführte Medikation im ausgestoßenen Zustand der Injektionsnadel über eine kommunizierende Verbindung zugeführt werden kann. Durch ein in der Katheterspitze vorgesehenes Zweikammersystem, in welchem koaxial ineinander zwei Kolben verschiebbar sind, kann einerseits über den einen Kolben die Injektionsnadel ausgestoßen, und andererseits über den zweiten Kolben eine bestimmte Dosierung der Medikation durch die Injektionsnadel appliziert werden.

In der unter Art. 54(3) zum Stand der Technik gehörenden WO-A-9408653 wurde bereits ein Katheter der eingangs erwähnten Art vorgeschlagen. Bei diesem Katheter werden mehrere Injektionsnadeln in einem Mehrlumenschaft längsverschoben, wobei die Katheterspitze über eine aufgesteckte und aufgeklebte Hülse mit dem Mehrlumenschaft verbunden ist. Eine entsprechende Offenbarung geht aus der GB-A-2269538 hervor.

Da der Katheter zur Behandlung von Koronargefäßen und -arterien verhältnismäßig lang sein muß, und in der Regel etwa 1,2 m bis 1,6 m lang sein kann, ergibt sich eine verhältnismäßig große Reibung, wenn eine Vielzahl von Injektionsnadeln über die gesamte Katheterlänge in den einzelnen Lumen des Mehrlumenschaftes verschoben werden müssen. Von Nachteil erweist sich auch, daß durch die verhältnismäßig hohe Reibung über die große Länge bei der Verwendung von Kunststoffen für den Mehrlumenschlauch unerwünschte Längsdehnungen oder Längsstauchungen auftreten können, die die genaue Positionierung der Injektionsnadeln im Gewebe erschweren.

Der Erfindung liegt die Aufgabe zugrunde, diese Nachteile zu überwinden.

Zur Lösung sieht die Erfindung gemäß Anspruch 1 vor, daß die zu einem Bündel zusammengefaßten Injektionsnadeln im Katheterschaft befestigt sind, und daß die Injektionsnadeln durch Verschieben der Katheterspitze relativ zum Katheterschaft die aus der Katheterspitze hervorstehende Position oder die eingezogene Position einnehmen.

Dadurch wird in vorteilhafter Weise erreicht, daß je nach Ausgestaltung des Katheterkopfes bzw. des Bündelns der Injektionsnadeln, die Oberflächenreibung stark verringert werden kann und dadurch ein präzises Positionieren der Injektionsnadel im Gewebe möglich ist.

Im Interesse einer sehr gefingen Reibung über die Länge des Katheters sieht eine weitere Ausgestaltung der Erfindung vor, daß der Katheterschaft ein Multilumenschaft ist, in dem die Injektionsnadeln in den einzelnen Lumina fixiert sind, und bei dem ein Betätigungsdraht zum Einziehen und Ausstoßen der Katheterspitze aus dem Katheterschaft durch ein Führungslumen im Katheterschaft verläuft.

Bei der Ausgestaltung der Erfindung gemäß dem unabhängigen Anspruch 4 ist vorgesehen, daß die zu einem Bündel zusammengefaßen Injektionsnadeln (15) nach dem distalen Bereich des Katheters in ein in seinem Innendurchmesser die Injektionsnadeln (15) aufnehmendes flexibles Rohr (24) innerhalb des Katheterschaft (14) eingesetzt sind.

Durch diese Ausgestaltung ergibt sich der Vorteil, daß nur zwei koaxiale rohrförmige Lumina vorhanden sind, die aufgrund ihrer Ausgestaltung, sei es aus geeigneten Kunststoffen oder Metallen, keine unerwünschte Längsdehnung oder Längsstauchung zeigen, und somit die Positionierung der Injektionsnadel mit geringer Kraft möglich machen. Dabei ist vorgesehen, daß das Bündel der Injektionsnadeln zwischen dem Katheterkopf und den koaxialen Lumina über eine Länge gebündelt verlaufen, die wegen der leichten Flexibilität der gebündelten Injektionsnadeln eine hohe Flexibilität des distalen Abschnitts des Katheters gewährleisten.

Um die distale Flexibilität des Katheters weiter zu erhöhen, ist auch vorgesehen, die einzelnen Injektionsnadeln in ihrem distalen Bereich bei gleichen Durchmesservolumen dünnwandiger auszuführen, wodurch sich dünnere und damit flexiblere Injektionsnadeln ergeben. Der dickwandigere Teil der Injektionsnadeln kann entweder als Bündel weiter über die gesamte Länge in einem rohrförmigen Außenschaft geführt sein, oder aber gemäß dem unabhängigen Anspruch 5 auch in ein rohrförmiges Innenlumen eingeklebt oder eingelötet sein, um mit einem größeren Durchmesservolumen bis zur Betätigungsvorrichtung weitergeführt zu werden. Dadurch wird erreicht, daß das zugeführte Fluid den Injektionsnadeln mit einem hohen Überschuß zugeführt wird, und dadurch gleichmäßig appliziert werden kann.

Zur Stabilisierung gegen unerwünschte Längsdehnung oder Längsstauchung kann auch, nach einer weiteren Ausgestaltung der Erfindung, in den Katheterschaft ein Edelstahl-Drahtgeflecht eingespritzt sein, oder in dem Katheterschaft bzw. in dem Multilumenbündel ein Stabilisierungsdraht eingebaut sein.

Zur Positionierung des Katheters bzw. zur gezielten Führung ist vorgesehen, daß in einem in das Bündel der Injektionsnadeln eingebundenen Führungslumen ein Führungsdraht geführt wird. Dieses Führungslumen kann auch zwischen dem Nadelbündel und dem Außenschaft des Katheters exzentrisch angeordnet sein, wobei der Führungsdraht extrakorporal vor der Betätigungsvorrichtung austritt. Bei dem Katheter ohne Führungslumen ist vorgesehen, daß an dem distalen Ende der Katheterspitze ein Führungsdraht befestigt ist.

Zur möglichst reibungslosen Führung der Injektionsnadeln in der Katheterspitze ist vorgesehen, daß die Katheterspitze aus einem Edelstahl-Rohrbündel besteht, durch dessen Lumina die Injektionsnadeln und ggf. ein Führungsdraht verläuft. Diese Ausführungsform der Katheterspitze ist besonders vorteilhaft für die Verwendung von vorgebogenen Injektionsnadeln, oder von einem Nadelmaterial, welches superelastische Eigenschaften und/oder shape-memory-Eigenschaften aufweist. Eine besonders vorteilhafte Führung der Injektionsnadeln in der Katheterspitze ergibt sich auch dadurch, daß das Bündel der Injektionsnadeln durch eine zentrale Bohrung in der Katheterspitze eingeführt und seitlich durch mit der zentralen Bohrung kommunizierende Führungsschlitze austritt.

Es ist offensichtlich, daß bei einer Vielzahl von Führungslumen in der Katheterspitze nicht alle mit Injektionsnadeln bestückt sein müssen. Vielmehr können einzelne Führungslumen auch für Lichtleiter benutzt werden, mit denen Lichtenergie durch den Katheter an das Gewebe herangeführt werden kann, um bei der Anwendung von photoaktivierbaren Substanzen die hierfür erforderliche Lichtenergie zuzuführen. Auch ist die Einführung von Kontroll- und/oder Meßsystemen vorgesehen. Bei einer solchen Ausgestaltung ist die Betätigungsvorrichtung entsprechend umzugestalten, damit die für die Lichtleiter vorgesehen Lumina durch die Betätigungsvorrichtung direkt zugänglich sind. Es ist jedoch auch vorgesehen, extrakorporal vor der Betätigungsvorrichtung die Führungslumina enden zu lassen, um derartige Lichtleiter oder auch einen Führungsdraht in den Katheter einführen zu können.

Die Betätigungsvorrichtung für einen derartigen Katheter hat einen Schubkolben für die Injektionsnadeln oder den Betätigungsdraht, wobei der Schubkolben mit einem Gewinde für die Axialverschiebung mit einer Rändelmutter versehen ist und an der Betätigungsvorrichtung mit den einzelnen Injektionsnadeln korrespondierende Medikations-Ports vorgesehen sind. Es ist jedoch auch möglich, die Vorschubbewegung des Schubkolbens pneumatisch, hydraulisch oder elektrisch zu steuern, indem entsprechende Antriebsmittel an der Betätigungsvorrichtung vorgesehen sind. Auch eine fußbetätigte Schubkolbenbetätigung kann vorgesehen sind. Derartige nichtmanuelle Vorschubbetätigungen sind insbesondere dann von Vorteil, wenn mehrere Medikations-Ports an der Betätigungsvorrichtung vorgesehen sind. Die Betätigungsvorrichtung ist zweckmäßiger Weise mit einer Skalierung versehen, damit ein definierter und kontrollierter Vorschubweg gewährleistet wird. Dabei kann durch Anschläge für eine definierte Begrenzung des Vorschubweges gesorgt werden.

Schließlich ist für besondere Anwendungsfälle auch vorgesehen, daß an dem Injektionskatheter distal und/oder auch proximal zur Katheterspitze sowohl ein Fixierballon zur Verankerung des Katheters während der Injektion als auch ein angioplastischer Ballon angebracht sein kann, um gleichzeitig mit der Injektion auch eine Dilatation durchführen zu können.

Die Injektionsnadeln können Lumina zum Einführen von Kontroll- und Meßgeräte dienen. Infrage kommen hierfür z.B. Angioskope, Ultraschallmeßgeräte, Spektroskope, Aktivitäts- und Konzentrations- sowie PH-Wertmeßgeräte oder Temperaturmeßgeräte und ähnliches. Selbstverständlich ist der Injektionskatheter auch zum Einsatz durch Punktionskanäle vorgesehen, um z.B. Tumore, Metastasen, Entzündungsherde, Konvolute oder ähnliches zu behandeln.

Die Vorteile und Merkmale der Erfindung ergeben sich auch aus der nachfolgenden Beschreibung von Ausführungsbeispielen in Verbindung mit den Ansprüchen und der Zeichnung. Es zeigen:
- Fig. 1: eine in einem Katheterschaft verschiebbare Katheterspitze zur Führung eines in mehreren Lumen des Katheterschaftes gehalterten Bündels von Injektionsnadeln beim Ein- und Ausfahren;
- Fig. 2: eine Katheterspitze mit eingezogenen Injektionsnadeln, welche als Bündel in einem Einlumenschaft gehaltert sind;
- Fig. 3: eine an einem Einlumenschaft fixierte Katheterspitze mit einem in dem Einlumenschaft verschiebbaren flexiblen Rohr, an welchem ein in der Katheterspitze verschiebbares Bündel von Injektionsnadeln befestigt ist;
- Fig. 4: eine als Rohrbündel ausgeführte Katheterspitze, welche an einem Einlumenschaft befestigt ist, durch welchen ein Bündel von Injektionsnadeln verschiebbar ist;
- Fig. 5: eine Betätigungsvorrichtung zum Verschieben eines Bündels von Injektionsnadeln in einem Einlumenschaft;
- Fig. 6: ein Schnitt durch die aus einem Rohrbündel bestehende Katheterspitze gemäß Fig. 4;
- Fig. 7: eine weitere Ausführungsform einer Katheterspitze;
- Fig. 8: einen Schnitt durch einen Katheterschaft mit Nadellumina und Führungslumina.

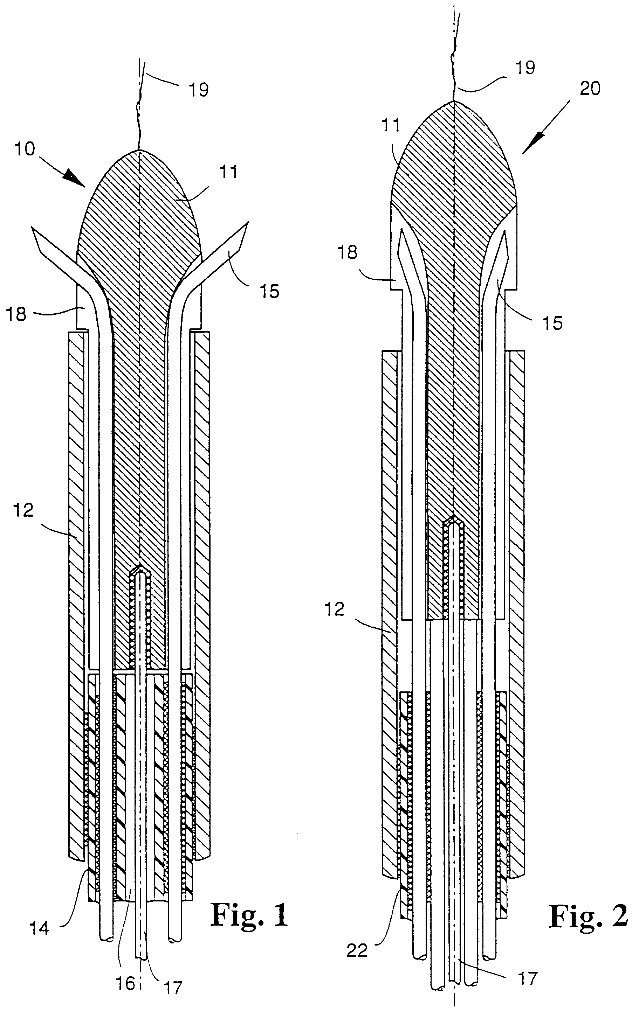

In Fig. 1 ist das distale Ende eines Katheters dargestellt, bei dem eine Katheterspitze 11 in einer Fixierhülse 12 axial verschiebbar angeordnet ist. Die Fixierhülse 12 ist mit einem Mehrlumenschaft 14 verbunden, bei dem in einzelnen Lumina Injektionsnadeln 15 befestigt sind. In einem zentralen Führungslumen 16 ist ein Betätigungsdraht 17 geführt, der in der Katheterspitze 11 verankert ist, und mit welchem die Katheterspitze in axialer Richtung verschoben werden kann. Bei der in Fig. 1 dargestellten eingezogenen Position der Katheterspitze sind die Injektionsnadeln 15 durch Führungsnuten 18 nach außen verschoben, in der sie in das den Katheter umgebende Gewebe einstechen können. Die Injektionsnadeln sind vorzugsweise derart ausgestaltet, daß sie eine gewisse Vorbiegung haben und dadurch leichter in der Führungsnut nach außen verschiebbar sind. Für das Nadelmaterial werden vorzugsweise Edelstähle oder Sonderlegierungen benutzt, die eine ausreichende Flexibilität und Steifigkeit haben, um einerseits durch die Wirkung der Katheterspitze nach außen verschoben werden zu können, die jedoch andererseits ausreichend starr sind, um in das Gewebe einzudringen. Um optimal in das Gewebe einzustechen, können verschiedene Anschliffarten der Nadeln Verwendung finden.

Die in Fig. 2 dargestellte Ausführungsform eines distalen Endes 20 eines Katheters zeigt eine Katheterspitze 11 in einer nach vorne herausgeschobenen Position, so daß die Injektionsnadeln 15 innerhalb der Führungsnuten 18 zu liegen kommen. In dieser Position wird der Katheter in die Arterie oder Vene eingeführt, wobei ein an seinem vorderen Ende als sogenannter fixed-wire angebrachter Führungsdraht 19 das Einführen des Katheters erleichtert. Bei dem Katheter gemäß Fig. 2 sind die Injektionsnadeln 15 zu einem Bündel zusammengefaßt und als solches in einem Einlumenschaft 22 befestigt. Der Betätigungsdraht 17 läuft im Innern des Bündels der Injektionsnadeln und wird dabei von diesen geführt. Er ist in der Katheterspitze 11 befestigt, so daß durch eine axiale Verschiebung des Betätigungsdrahtes 17 die Katheterspitze aus dem distalen Ende des Katheters herausverschoben oder hineingezogen werden kann.

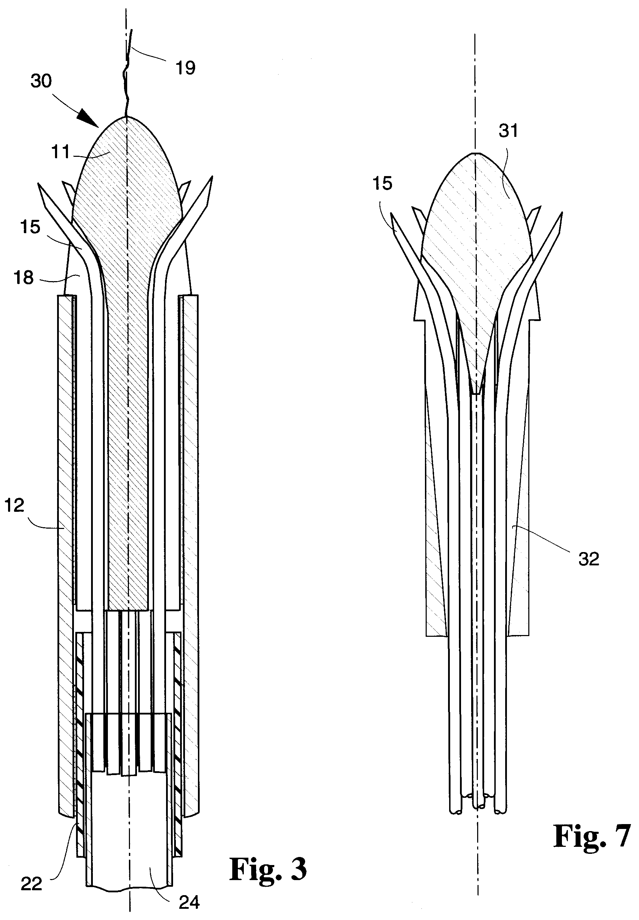

In Fig. 3 ist eine weitere Ausgestaltung der Erfindung dargestellt, bei der das Bündel der Injektionsnadeln innerhalb des Einlumenschaftes 22 längsverschiebbar ist, und die Katheterspitze mit Hilfe der Fixierhülse 12 am Einlumenschaft 22 in einer unverschiebbaren Position festgehalten wird. Innerhalb des Einlumenschaftes 22 ist ein flexibles Rohr 24 vorgesehen, in dessen vorderes Ende die rückwärtigen Abschnitte der Injektionsnadeln 15 eingelötet oder eingeklebt sind, so daß die Injektionsnadeln durch ein axiales Verschieben des flexiblen Rohres 24 im Einlumenschaft 22 in die Katheterspitze eingezogen bzw. durch diese nach außen verschoben werden können. Der Abstand zwischen der Katheterspitze 11 und dem flexiblen Rohr kann wesentlich länger sein als in der Darstellung gezeigt, damit eine hohe Flexibilität des distalen Endes gewährleistet ist. Das heißt, abweichend von der Darstellung greift die Fixierhülse 12 nicht bis zu einem Bereich in dem sich das flexible Rohr 24 bewegt. Um die Verschiebbarkeit zu erleichtern, ist auch vorgesehen, daß das flexible Rohr 24 mit einer Gleitbeschichtung versehen ist, die z.B. aus PTFE bestehen kann. Dasselbe gilt auch für die im Mehrlumenschaft gemäß Fig. 1, bzw. im Einlumenschaft gemäß Fig. 2 verschiebbaren Injektionsnadeln.

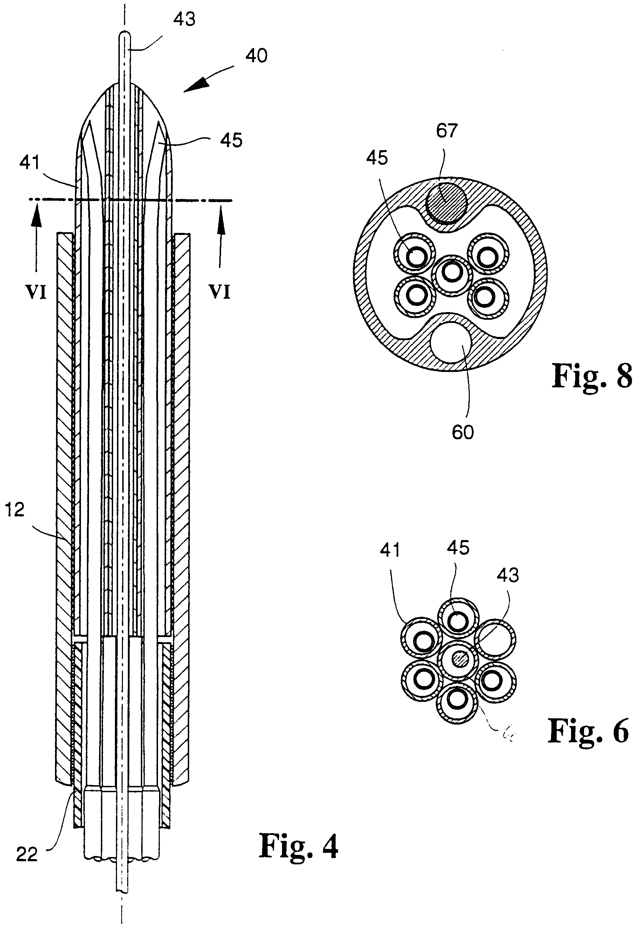

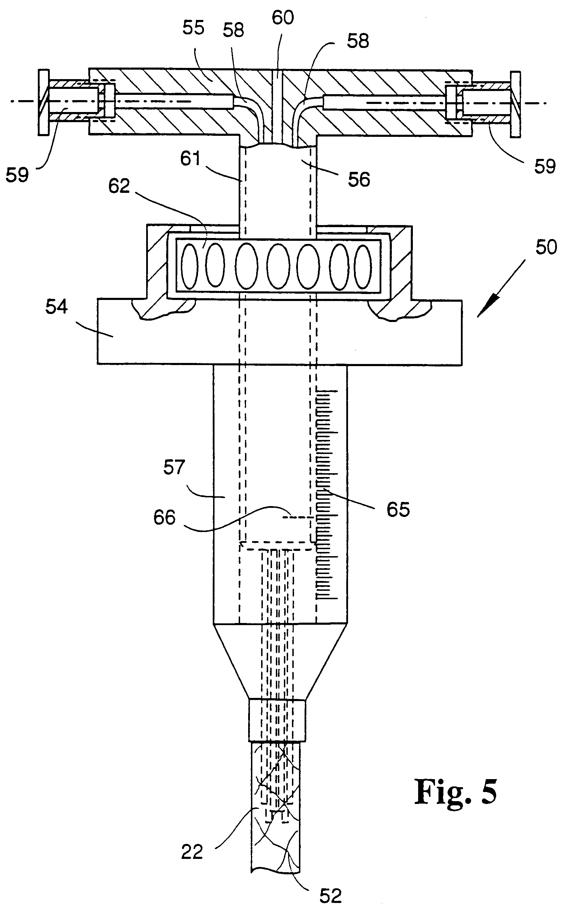

Fig. 4 zeigt eine weitere Ausführungsform der Erfindung, bei der das distale Ende 40 des Katheters eine aus einem Rohrbündel 41 bestehende Katheterspitze aufweist, deren Querschnitt aus Fig. 6 hervorgeht. Die vorgebogenen Injektionsnadeln verlaufen innerhalb der einzelnen Rohre des Rohrbündels bis zu einem Einlumenschlauch 22, der über die Fixierhülse 12 mit der Katheterspitze fest verbunden ist. Der Durchmesser der Injektionsnadeln 45 ist im Bereich der Katheterspitze sowie im Bereich des Einlumenschaftes 22 dünner als in dem restlichen Bereich des Katheters, wodurch eine hohe Flexibilität der vorderen Enden der Injektionsnadeln erreicht wird, und im Bereich des Katheterschaftes eine optimale Steifigkeit sowie Flexibilität gewährleistet ist. Die vorzugsweise gleichbeschichteten Injektionsnadeln gleiten innerhalb des Einlumenschaftes 22 und geben vorzugsweise in ihrer Mitte ein Lumen für einen Führungsdraht 43 frei, der durch ein entsprechendes Rohr im Rohrbündel der Katheterspitze nach vorne austreten kann. Die durchmessergrößere Verlängerung der Injektionsnadeln 45 kann auch mit einem größeren Innendurchmesser als die Spitzen der Injektionsnadeln versehen sein, um die Durchflußreibung herabzusetzen und das Medikament mit geringerem Druck injizieren zu können. Auch bei dieser Darstellung ist der Abstand der durchmessergrößeren Abschnitte der Injektionsnadeln von der Katheterspitze verhältnismäßig kurz dargestellt. Im Interesse einer erhöhten Flexibilität der Katheterspitze ist es zweckmäßig die verdickten Abschnitte erst in einem größeren Abstand von der Fixierhülse 12 beginnen zu lassen.

Zur axialen Verschiebung der Injektionsnadeln kann eine Betätigungsvorrichtung Verwendung finden, wie sie in Fig. 5 dargestellt ist. Diese Betätigungsvorrichtung besteht im wesentlichen aus zwei Teilen 54 und 55, wobei der Teil 54 als feststehend und der Teil 55 als beweglich bezeichnet wird. Der bewegliche Teil 55 ist mit einem Schubkolben 56 versehen, der in einen Zylinder 57 eintaucht, und in welchem die extrakorporalen Enden der Injektionsnadeln befestigt sind, die über Bohrungen 58 mit Medikationsanschlüssen 59 verbunden sind. Ferner verläuft durch den beweglichen Teil 55 eine Bohrung 60, die in das Führungsvolumen im Katheterschaft übergeht. Auf dem Schubkolben 56 ist ein Gewinde angeordnet, auf welches eine Rändelmutter 62 aufgeschraubt ist, die am feststehenden Teil 54 geführt gehaltert wird, so daß durch Drehen dieser Rändelschraube der Schubkolben mehr oder weniger in den Zylinder 57 hineingedrückt und damit die Injektionsnadeln verschoben werden können. Am Zylinder 57 kann eine Skala 65 und am Schubkolben 56 ein Meßstrich 66 angebracht sein, um das Maß der Verschiebung feststellen und kontrollieren zu können. Mit dem Zylinder 57 ist der Schaft des Katheters verbunden, in welchen ein Edelstahl-Drahtgeflecht eingespritzt sein kann, um den Schaft gegen eine unerwünschte Längsdehnung oder Längsstauchung beim Verschieben der Injektionsnadeln zu stabilisieren. In der dargestellten Form ist die Betätigungsvorrichtung insbesondere für einen Katheter verwendbar, der ein distales Ende gemäß Fig. 4 hat.

Bei einem Katheter mit einem distalen Ende gemäß Fig. 3 ist das flexible Rohr 24 anstelle des Bündels der Injektionsnadeln am Schubkolben 56 befestigt, und außerdem nur ein Medikationsanschluß 59 vorgesehen, der mit dem Innern des flexiblen Rohres 24 in Verbindung steht.

Für einen Katheter mit einem distalen Ende gemäß den Fig. 1 und 2, sind die extrakorporalen Enden der Injektionsnadeln mit dem feststehenden Teil 54 der Betätigungsvorrichtung verbunden, der auch mit entsprechenden Anschlüssen für die Medikation versehen ist. Der bewegliche Teil 55 der Betätigungsvorrichtung wirkt auf den Betätigungsdraht 17, mit dem die Katheterspitze am distalen Ende in Längsrichtung verschoben werden kann.

Obwohl in der Zeichnung nicht dargestellt, ist auch vorgesehen, daß das Führungslumen für den Führungsdraht nicht an der Betätigungsvorrichtung austritt, sondern vor dieser im extrakorporalen Bereich des Katheterschaftes, zu welchem Zweck das Führungslumen zwischen dem Nadelbündel und dem Außenschaft exzentrisch geführt ist.

Schließlich ist auch vorgesehen, daß mit dem Schaft des Katheters ein Stabilisierungsdraht verbunden sein kann, der entweder in den Außenmantel des Schaftes eingespritzt, oder bei einem mehrlumigen Schaft in einem Lumen vorzugsweise durch Kleben befestigt ist. Bei dem in Fig. 8 dargestellten Schnitt durch einen Katheter mit einem Einlumenschaft, sind beispielweise die beiden Lumen für den Führungsdraht und den Stabilisierungsdraht in den Umfang des Schaftes mit eingespritzt, wogegen das Bündel der Injektionsnadeln im Innern des Schaftes frei verschiebbar angeordnet ist.

Für die Katheterspitze können unterschiedliche Materialien Verwendung finden, und zwar sowohl Kunststoff als auch Metall, Keramik oder Glas, wobei es wünschenswert sein kann, daß die Katheterspitze röntgendicht ausgeführt ist.

Ferner ist vorgesehen, daß die im Katheter verschiebbaren Lumen innerhalb des Katheters gegen rückfließende Flüssigkeiten abgedichtet sind, wobei z.B. geeignete Gleitmittel diese Funktion übernehmen können.

Zur Stabilisierung des Katheterschaftes und um ein gleichmäßiges Austreten der Injektionsnadel aus der Katheterspitze zu gewährleisten, ist auch vorgesehen, den Katheterschaft zu verdrillen, bzw. das Bündel der Injektionsnadeln im Katheterschaft längs einer langgezogenen Schraubenlinie zu ordnen. Dadurch läßt sich die Verschiebbarkeit erleichtern, insbesondere in Verbindung mit reibungsreduzierenden Gleitmitteln.

Der Funktionseinsatz des Injektionskatheters kann dadurch optimiert werden, daß an dem Katheter in bereits vorgeschlagener Weise Ballons angebracht werden, die den Katheter während der Injektion fixieren. Auch angioplastische Ballons, die sowohl distal als auch proximal zur Katheterspitze angeordnet sind, verbessern die Einsetzbarkeit des Katheters und können insbesondere dazu beitragen, daß gleichzeitig mit einer Ballondilatation eine Injektion eines Arzneimittels in dem betroffenen Gewebe ausgeführt werden kann.

## Patentansprüche

1. Katheter zur Injektion eines Fluids bzw. eines Arzneimittels in Hohlorgane und Körperhöhlen, insbesondere in Koronargefäße und Arterien mit einer in Arterien einführbaren Katheterspitze (11) und einem Katheterschaft (12,14;12,22), mehreren in der Katheterspitze verschiebbar angeordneten Injektionsnadeln (15), wobei die Nadelspitzen in einer eingezogenen Position innerhalb der Katheterspitze liegen und für die Applikation des Fluids oder Arzneimittels in einer herausgeschobenen Position aus der Katheterspitze herausstehen, einer am extrakorporalen Ende angebrachten Betätigungsvorrichtung (50), mit in der Katheterspitze vorgesehenen Öffnungen (18) mit Nadelführungen, die sowohl einen nach seitlich als auch nach vorne gerichteten Austritt der Injektionsnadeln zulassen, wobei der Katheterschaft (12,14;12,22) am einen Teil (57) der Betätigungsvorrichtung befestigt ist und die Katheterspitze (11) mit einem anderen Teil (56) der Betätigungsvorrichtung verbunden ist, wodurch mehrere der parallel geführten Injektionsnadeln (15) beim Betätigen der Betätigungsvorrichtung von der zurückgezogenen in die nach außen vorstehende Position durch eine relative Verschiebung der Katheterspitze und der Injektionsnadeln zueinander bewegbar sind,
dadurch gekennzeichnet,
daß die zu einem Bündel zusammengefaßten Injektionsnadeln (15) im Katheterschaft (12,14;12,22) befestigt sind, und daß die Injektionsnadeln durch Verschieben der Katheterspitze (11) relativ zum Katheterschaft die aus der Katheterspitze (11) hervorstehende Position oder die eingezogene Position einnehmen.

2. Katheter nach Anspruch 1,
dadurch gekennzeichnet,
daß der Katheterschaft (14) ein Multilumenschaft ist, in dem die Injektionsnadeln (15) in den einzelnen Lumina fixiert sind, und bei dem ein Betätigungsdraht (17) zum Einziehen und Ausstoßen der Katheterspitze (11) aus dem Katheterschaft durch ein Führungslumen (16) im Katheterschaft verläuft.

3. Katheter nach Anspruch 1,
dadurch gekennzeichnet,
daß der Katheterschaft (14) ein Einlumenschaft (22) ist, in dem die gebündelten Injektionsnadeln (15) im Innern den mit der Katheterspitze (11) verbundenen Betätigungsdraht (17) führen.

4. Katheter zur Injektion eines Fluids bzw. eines Arzneimittels in Hohlorgane und Körperhöhlen, insbesondere in Koronargefäße und Arterien mit einer in Arterien einführbaren Katheterspitze (11) und einem Katheterschaft (12,14), mehreren in der Katheterspitze verschiebbar angeordneten Injektionsnadeln (15), wobei die Nadelspitzen in einer eingezogenen Position innerhalb der Katheterspitze liegen und für die Applikation des Fluids oder Arzneimittels in einer herausgeschobenen Position aus der Katheterspitze herausstehen, einer am extrakorporalen Ende angebrachten Betätigungsvorrichtung (50), mit in der Katheterspitze vorgesehenen Öffnungen (18) mit Nadelführungen, die sowohl einen nach seitlich als auch nach vorne gerichteten Austritt der Injektionsnadeln zulassen, wobei der Katheterschaft (12,14) am einen Teil (57) der Betätigungsvorrichtung befestigt ist und die Katheterspitze (11) mit einem anderen Teil (56) der Betätigungsvorrichtung verbunden ist, wodurch mehrere der parallel geführten Injektionsnadeln (15) beim Betätigen der Betätigungsvorrichtung von der zurückgezogenen in die nach außen vorstehende Position durch eine relative Verschiebung der Katheterspitze und der Injektionsnadeln zueinander bewegbar sind,
dadurch gekennzeichnet,
daß die zu einem Bündel zusammengefaßen Injektionsnadeln (15) proximal vom distalen Bereich des Katheters in ein in seinem Innendurchmesser die Injektionsnadeln (15) aufnehmendes flexibles Rohr (24) innerhalb des Katheterschaftes (14) eingesetzt sind.

5. Katheter zur Injektion eines Fluids bzw. eines Arzneimittels in Hohlorgane und Körperhöhlen, insbesondere in Koronargefäße und Arterien mit einer in Arterien einführbaren Katheterspitze (11) und einem Katheterschaft (12,14), mehreren in der Katheterspitze verschiebbar angeordneten Injektionsnadeln (15), wobei die Nadelspitzen in einer eingezogenen Position innerhalb der Katheterspitze liegen und für die Applikation des Fluids oder Arzneimittels in einer herausgeschobenen Position aus der Katheterspitze herausstehen, einer am extrakorporalen Ende angebrachten Betätigungsvorrichtung (50), mit in der Katheterspitze vorgesehenen Öffnungen (18) mit Nadelführungen, die sowohl einen nach seitlich als auch nach vorne gerichteten Austritt der Injektionsnadeln zulassen, wobei der Katheterschaft (12,14) am einen Teil (57) der Betätigungsvorrichtung befestigt ist und die Katheterspitze (11) mit einem anderen Teil (56) der Betätigungsvorrichtung verbunden ist, wodurch mehrere der parallel geführten Injektionsnadeln (15) beim Betätigen der Betätigungsvorrichtung von der zurückgezogenen in die nach außen vorstehende Position durch eine relative Verschiebung der Katheterspitze und der Injektionsnadeln zueinander bewegbar sind,
dadurch gekennzeichnet,
daß der Katheterschaft ein Einlumenschaft (22) ist, in dessen Innern die zu einem Bündel zusammengefaßten Injektionsnadeln (45) relativ zum Katheterschaft derart verschiebbar sind, daß die Injektionsnadeln (45) eine aus der Katheterspitze (41) hervorstehende oder eingezogene Position einnehmen.

6. Katheter nach Anspruch 1, 2 oder 5,
dadurch gekennzeichnet,
daß die Injektionsnadeln (15; 45) in ihrem distalen Bereich bei gleichem Durchflußvolumen dünnwandiger ausgeführt sind.

7. Katheter nach Anspruch 5,
dadurch gekennzeichnet,
daß ein Führungsdraht (43) zwischen dem von den Injektionsnadeln (45) gebildeten Bündel und dem Katheterschaft exzentrisch geführt ist, sowie extrakorporal vor der Betätigungsvorrichtung (50) austritt.

8. Katheter nach Anspruch 2
dadurch gekennzeichnet,
daß der Betätigungsdraht (17) durch ein Führungslumen (60) im Katheterschaft zum Einziehen und Ausstoßen der Katheterspitze (11) aus dem Katheterschaft verläuft.

9. Katheter nach einem oder mehreren der Ansprüche 1 bis 8,
dadurch gekennzeichnet,
daß die Katheterspitze (41) aus einem Edelstahl-Rohrbündel besteht, durch dessen Lumen die Injektionsnadeln (45) und ggf. ein Führungsdraht (43) verläuft.

10. Katheter nach einem der Ansprüche 1 bis 9,
dadurch gekennzeichnet,
daß das von den Injektionsnadeln (15) gebildete Bündel durch eine zentrale Bohrung (32) der Katheterspitze (31) eingeführt ist, und seitlich durch mit der zentralen Bohrung kommunizierende Führungsnuten (18) austritt.

11. Katheter nach einem oder mehreren der Ansprüche 1 bis 10,
dadurch gekennzeichnet,
daß in den Katheterschaft ein Edelstahl-Drahtgeflecht (52) eingespritzt ist.

12. Katheter nach einem oder mehreren der Ansprüche 1 bis 11,
dadurch gekennzeichnet,
daß in den Katheterschaft ein Stabilisierungsdraht (67) eingebaut ist.

13. Katheter nach einem oder mehreren der Ansprüche 1 bis 12,
dadurch gekennzeichnet,
daß die Betätigungsvorrichtung (50) einen Schubkolben (56) für das Verschieben des Betätigungsdrahtes (17) hat,
daß der Schubkolben (56) mit einem Gewinde (61) für die Axialverschiebung mit einer Rändelmutter (62) versehen ist,
und daß in der Betätigungsvorrichtung mit den einzelnen Injektionsnadeln (15) kommunizierende Medikationsanschlüsse (59) vorgesehen sind.

## Claims

1. A catheter for injecting a fluid or medication into hollow organs and body cavities, particularly into coronary vessels and arteries, comprising: a catheter tip (11) adapted to be inserted into arteries; a catheter stem (12,14;12,22); a plurality of injection needles (15) arranged in the catheter tip for movement therein, said injection needles having needle points which, in a retracted position, lie inside the catheter tip and, in an extended position, lie exposed in readiness for applying said fluid or medicine; an operating device (50) connected to the extracorporal end of the catheter; openings (18) formed in the catheter tip and allowing the needles points to protrude therefrom laterally as well as forwardly; said catheter stem (12,14;12,22) being connected to one part (57) of the operating device and the injection needles (11) being connected to the other part (56) thereof; the arrangement being such that movement of one of said two parts in a predetermined direction relative to the other effects a corresponding relative movement between the catheter tip and the plurality of injection needles (15), thereby causing the needle points to be moved from the retracted position to the extended position thereof, said injection needles being joined together as a bundle displaceable relative to the catheter tip,
characterized in that
the injection needles joined together as a bundle are connected to the catheter stem (12,14;12,22) and that the injection needles by movement of the catheter tip (11) relative to the catheter stem are positioned in said extended position or said retracted position.

2. A catheter according to claim 1, wherein the catheter stem (14) is a multi-lumen stem having injection needles (15) secured within the various lumina, and wherein an operating wire (17) is guided within a guide lumen (16) of said catheter stem for retracting and extending of the catheter tip (11).

3. A catheter according to claim 1, wherein the catheter stem (14) is a single-lumen stem (22) within which the bundle of injection needles (15) guides, in its inside, the guide wire (17) connected to the catheter tip (11).

4. A catheter for injecting a fluid or medication into hollow organs and body cavities, particularly into coronary vessels and arteries, comprising: a catheter tip (11) adapted to be inserted into arteries; a catheter stem (12,14); a plurality of injection needles (15) arranged in the catheter tip for movement therein, said injection needles having needle points which, in a retracted position, lie inside the catheter tip and, in an extended position, lie exposed in readiness for applying said fluid or medicine; an operating device (50) connected to the extracorporal end of the catheter; openings (18) formed in the catheter tip and allowing the needles points to protrude therefrom laterally as well as forwardly; said catheter stem (12,14) being connected to one part (57) of the operating device and the injection needles (11) being connected to the other part (56) thereof; the arrangement being such that movement of one of said two parts in a predetermined direction relative to the other effects a corresponding relative movement between the catheter tip and the plurality of injection needles (15), thereby causing the needle points to be moved from the retracted position to the extended position thereof; said injection needles being joined together as a bundle displaceable relative to the catheter tip,
characterized in that
the injection needles (15) joined together as a bundle are inserted, behind the distal region of the catheter, into a flexible tube (24) receiving the injection needles (15) within the inside diameter of the catheter stem (14).

5. A catheter for injecting a fluid or medication into hollow organs and body cavities, particularly into coronary vessels and arteries, comprising: a catheter tip (11) adapted to be inserted into arteries; a catheter stem (12,14); a plurality of injection needles (15) arranged in the catheter tip for movement therein, said injection needles having needle points which, in a retracted position, lie inside the catheter tip and, in an extended position, lie exposed in readiness for applying said fluid or medicine; an operating device (50) connected to the extracorporal end of the catheter; openings (18) formed in the catheter tip and allowing the needles points to protrude therefrom laterally as well as forwardly; said catheter stem (12,14) being connected to one part (57) of the operating device and the injection needles (11) being connected to the other part (56) thereof; the arrangement being such that movement of one of said two parts in a predetermined direction relative to the other effects a corresponding relative movement between the catheter tip and the plurality of injection needles (15), thereby causing the needle points to be moved from the retracted position to the extended position thereof, said injection needles being joined together as a bundle displaceable relative to the catheter tip,
characterized in that
the catherter stem is a single-lumen stem (22) within which the injection needles (45), joined together as a bundle, are movable relative to the catheter stem in such a way that the injection needles (45) are positioned either in the extended or retracted position.

6. A catheter according to claims 1, 2 or 5, wherein the injection needles (15;45) have a reduced wall thickness, but the same flow cross-section, in the distal region thereof.

7. A catheter according to claim 5, wherein a guide wire (43) is guided eccentrically between said bundle of injection needles (45) and the catheter stem and protrudes outwardly at an extracorporal location before the operating device (50).

8. A catheter according to claim 2,
characterized in that
the operating wire (17) extends through a guide lumen (60) within the catheter stem for retracting the catheter tip (11) respectively into and from the catheter stem

9. A catheter according to one or several of claims 1 to 8,
characterized in that
the catheter tip (41) consists of a bundle of stainless-steel tubes defining lumina which having the injection needles (45) and a guide wire (43) extending therethrough.

10. A catheter according to anyone of claims 1 to 9,
characterized in that
the bundle of injection needles (15) extends through a central bore (32) of the catheter tip and being laterally extendible through said guide grooves (18).

11. A catheter according to one or several of claims 1 to 10.
characterized in that
the catheter stem has a stainless-steel wire mesh (52) molded therein.

12. A catheter according to one or several of claims 1 to 11,
characterized in that
said catheter stem has a stabilizing wire (67) incorporated therein.

13. A catheter according to one or several of claims 1 to 12,
characterized in that
said operating device (50) includes a thrust plunger (56) for effecting discplacement of said operating wire (17),
said thrust plunger (56) having a threaded portion (61) which is threadedly engaged with a knurled nut (62) operable to effect axial displacement of the thrust plunger,
and said operating divide including medication inlets (59) which communicate with the individual injection needles (15).

## Revendications

1. Cathéter pour injecter un fluide ou un médicament dans des organes creux et des cavités du corps humain, notamment dans des vaisseaux sanguins coronaires et des artères, avec une pointe de cathéter (11) pouvant être introduite dans des artères et une tige de cathéter (12, 14 ; 12, 22), avec plusieurs aiguilles d'injection (15) disposées à coulissement dans la pointe de cathéter, les aiguilles d'injection se trouvant dans une position rentrée à l'intérieur de la pointe de cathéter et dépassant hors de la pointe de cathéter dans une position poussée vers l'extérieur en vue de l'application du fluide ou médicament, avec un dispositif d'actionnement (50) disposé à l'extrémité extracorporelle, avec des ouvertures (18) prévues dans la pointe de cathéter et pourvues de guides d'aiguille, qui autorisent une sortie des aiguilles d'injection dirigée tant sur les côtés que vers l'avant, la tige de cathéter (12, 14 ; 12, 22) étant fixée sur une partie (57) du dispositif d'actionnement et la pointe de cathéter (11) étant assemblée à une autre partie (56) du dispositif d'actionnement, de sorte que plusieurs des aiguilles d'injection (15) guidées en parallèle peuvent, en actionnant le dispositif d'actionnement, être déplacées de leur position rentrée dans leur position dépassant vers l'extérieur par un déplacement relatif entre la pointe de cathéter et les aiguilles d'injection,
**caractérisé** en ce que les aiguilles d'injection (15), réunies en un faisceau, sont fixées dans la tige de cathéter (12, 14 ; 12, 22), et en ce que les aiguilles d'injection prennent leur position dépassant hors de la pointe de cathéter (11) ou leur position rentrée par déplacement de la pointe de cathéter (11) par rapport à la tige de cathéter.

2. Cathéter selon la revendication 1, **caractérisé** en ce que la tige de cathéter (14) est une tige à lumières multiples, les aiguilles d'injection (15) étant fixées dans les lumières individuelles et un fil métallique d'actionnement (17), afin de rentrer et d'expulser la pointe de cathéter (11), s'étendant hors de la tige de cathéter à travers une lumière de guidage (16) prévue dans la tige de cathéter.

3. Cathéter selon la revendication 1, **caractérisé** en ce que la tige de cathéter (14) est une tige à une seule lumière (22), dans laquelle les aiguilles d'injection (15) réunies en faisceau guident, à l'intérieur du faisceau, le fil métallique d'actionnement (17) relié à la pointe de cathéter (11).

4. Cathéter pour injecter un fluide ou un médicament dans des organes creux et des cavités du corps humain, notamment dans des vaisseaux sanguins coronaires et des artères, avec une pointe de cathéter (11) pouvant être introduite dans des artères et une tige de cathéter (12, 14), avec plusieurs aiguilles d'injection (15) disposées à coulissement dans la pointe de cathéter, les aiguilles d'injection se trouvant dans une position rentrée à l'intérieur de la pointe de cathéter et dépassant hors de la pointe de cathéter dans une position poussée vers l'extérieur en vue de l'application du fluide ou médicament, avec un dispositif d'actionnement (50) disposé à l'extrémité extracorporelle, avec des ouvertures (18) prévues dans la pointe de cathéter et pourvues de guides d'aiguille, qui autorisent une sortie des aiguilles d'injection dirigée tant sur les côtés que vers l'avant, la tige de cathéter (12, 14) étant fixée sur une partie (57) du dispositif d'actionnement et la pointe de cathéter (11) étant assemblée à une autre partie (56) du dispositif d'actionnement, de sorte que plusieurs des aiguilles d'injection (15) guidées en parallèle peuvent, en actionnant le dispositif d'actionnement, être déplacées de leur position rentrée dans leur position dépassant vers l'extérieur par un déplacement relatif entre la pointe de cathéter et les aiguilles d'injection,
**caractérisé** en ce que les aiguilles d'injection (15) réunies en un faisceau sont installées à l'intérieur de la tige de cathéter (14), proximalement à la région distale du catéther, dans un tube flexible (24) qui reçoit les aiguilles d'injection (15) dans son diamètre intérieur.

5. Cathéter pour injecter un fluide ou un médicament dans des organes creux et des cavités du corps humain, notamment dans des vaisseaux sanguins coronaires et des artères, avec une pointe de cathéter (11) pouvant être introduite dans des artères et une tige de cathéter (12, 14), avec plusieurs aiguilles d'injection (15) disposées à coulissement dans la pointe de cathéter, les aiguilles d'injection se trouvant dans une position rentrée à l'intérieur de la pointe de cathéter et dépassant hors de la pointe de cathéter dans une position poussée vers l'extérieur en vue de l'application du fluide ou médicament, avec un dispositif d'actionnement (50) disposé à l'extrémité extracorporelle, avec des ouvertures (18) prévues dans la pointe de cathéter et pourvues de guides d'aiguille, qui autorisent une sortie des aiguilles d'injection dirigée tant sur les côtés que vers l'avant, la tige de cathéter (12, 14) étant fixée sur une partie (57) du dispositif d'actionnement et la pointe de cathéter (11) étant assemblée à une autre partie (56) du dispositif d'actionnement, de sorte que plusieurs des aiguilles d'injection (15) guidées en parallèle peuvent, en actionnant le dispositif d'actionnement, être déplacées de leur position rentrée dans leur position dépassant vers l'extérieur par un déplacement relatif entre la pointe de cathéter et les aiguilles d'injection,
**caractérisé** en ce que la tige de cathéter est une tige à une seule lumière (22), à l'intérieur de laquelle les aiguilles d'injection (45), réunies en un faisceau, peuvent être déplacées par rapport à la tige de cathéter de telle sorte que les aiguilles d'injection (45) prennent une position rentrée ou dépassant hors de la pointe de cathéter (41).

6. Cathéter selon la revendication 1, 2 ou 5, **caractérisé** en ce que les aiguilles d'injection (15 ; 45) sont réalisées à paroi plus mince dans leur région distale, avec le même volume de passage.

7. Cathéter selon la revendication 5, **caractérisé** en ce qu'un fil métallique de guidage (43) est guidé excentriquement entre le faisceau formé par les aiguilles d'injection (45) et la tige de cathéter, et sort extracorporellement à l'avant du dispositif d'actionnement (50).

8. Cathéter selon la revendication 2, **caractérisé** en ce que le fil métallique d'actionnement (17) s'étend à travers une lumière de guidage (60) dans la tige de cathéter afin de rentrer la pointe de cathéter (11) et de l'expulser hors de la tige de cathéter.

9. Cathéter selon une ou plusieurs des revendications 1 à 8, **caractérisé** en ce que la pointe de cathéter (41) consiste en un faisceau de tubes en acier spécial, à travers les lumières duquel s'étendent les aiguilles d'injection (45) et, le cas échéant, un fil métallique de guidage (43).

10. Cathéter selon une des revendications 1 à 9, **caractérisé** en ce que le faisceau formé par les aiguilles d'injection (15) est introduit par un perçage central (32) de la pointe de cathéter (31), et sort latéralement par des rainures de guidage (18) qui communiquent avec le perçage central.

11. Cathéter selon une ou plusieurs des revendications 1 à 10, **caractérisé** en ce qu'une tresse métallique (52) en acier spécial est injectée dans la tige de cathéter.

12. Cathéter selon une ou plusieurs des revendications 1 à 11, **caractérisé** en ce qu'un fil métallique stabilisateur (67) est incorporé dans la tige de cathéter.

13. Cathéter selon une ou plusieurs des revendications 1 à 12, **caractérisé** en ce que le dispositif d'actionnement (50) possède un piston pousseur (56) pour le déplacement du fil métallique d'actionnement (17),
en ce que le piston pousseur (56) est pourvu d'un filetage (61) pour le déplacement axial d'un écrou moleté (62),
et en ce que des raccords d'alimentation en médicaments (59), qui communiquent avec les aiguilles d'injection individuelles (15), sont prévus dans le dispositif d'actionnement.
